# EUROPEAN PATENT APPLICATION

(11) **EP 2 022 405 A1**
(43) Date of publication of application: **11.02.2009**
(21) Application number: 08013692.2
(22) Date of filing: 30.07.2008
(51) Int. Cl.: A61B 10/02, A61B 17/34

(54) **Bone marrow biopsy cannula**

(30) Priority: 31.07.2007 JP 2007199178
(71) Applicant: GC Corporation, Itabashi-ku Tokyo 174-8585 (JP)
(72) Inventor: Yamamoto, Katsushi, Itabashi-ku Tokyo 174-8585 (JP); Sakai, Yuhiro, Itabashi-ku Tokyo 174-8585 (JP)
(74) Representative: Müller-Boré & Partner Patentanwälte

(57) **Abstract**

To provide a cannula which is properly used for a bone marrow biopsy needle, can be operated easily to pierce a bone cortex of a bone tissue with weak force, and gives high airtightness in a state of piercing a bone cortex of a bone tissue, the cannula comprises a support cylinder (1) having an insertion port (1a) for inserting a stylet (S) and being fixed at a rear end of the cannula, a male screw part (2) is formed on an outer periphery of a tip of the cannula, and a cutting edge (2a), which is made as a recess toward a center axis and has a self-tapping function at one-side end edge of the recess, is formed along an axial direction at the tip side of the male screw part (2).

## Description

The present invention relates to a cannula having a support cylinder which is fixed at a rear end thereof and has an insertion port for inserting a stylet. This cannula is properly used as a bone marrow biopsy needle for a hard bone tissue such as a jawbone.

A cannula is generally used as a bone marrow biopsy needle in combination with a stylet inserted into an insertion port of a support cylinder fixed at a rear end of the cannula. When a bone marrow sample is collected from a bone tissue or various kinds of medical agents are injected into a bone marrow of a bone tissue by using such a cannula, the following steps are carried out. The cannula is thrust to pierce a bone cortex of a bone tissue until a tip thereof reaches a bone marrow of a bone tissue, after the stylet is inserted at first into the insertion port of the support cylinder fixed at a rear end of the cannula. Then, the bone marrow sample can be collected from the bone tissue by steps of removing the stylet, attaching a suction implement or an injector to the insertion port of the support cylinder of the cannula, and sucking. Alternatively, the bone marrow sample can be collected by steps of removing the stylet and inserting further the cannula into the bone marrow so as to make the bone marrow to enter the inside of the cannula. Besides, the bone marrow sample can be collected by steps of removing the stylet and inserting a collection implement from the insertion port of the support cylinder of the cannula. On the other hand, various kinds of medical agents can be injected into a bone marrow of a bone tissue by steps of removing the stylet attaching an injector containing an agent to the insertion port of the support cylinder of the cannula and injecting the agent.

For example, Japanese Translation of PCT Publication 2003-532462 discloses a cannula which is fixed on a handle and has a cutting edge at a tip thereof. Such a conventional general cannula for a bone marrow biopsy needle is used in a manner of thrusting the cannula to pierce a bone cortex of a bone tissue until the tip thereof reaches the bone marrow of the bone tissue, after the stylet is inserted into an insertion port of a support cylinder fixed at a rear end of the cannula. However, in this case, since the bone cortex of the bone tissue is very hard, there is a fault that it is necessary to press the handle of the cannula with strong force in order to make the tip of the cannula pierce the bone cortex of the bone tissue. Particularly, when the bone tissue to be pierced has a very hard bone cortex, such as a jawbone, the handle of the cannula should be pressed with all one's strength, and thus there is a fault that an operation is difficult. Further, there is also a fault that the cannula is deformed during an operation, or broken in the worst case.

Instead of the above method that the general cannula for a bone marrow biopsy needle is thrust to pierce a bone cortex of a bone tissue until the tip of the cannula reaches the bone marrow of the bone tissue by pressing the handle of the cannula with strong force so as to make the tip of the cannula pierce the bone cortex of the bone tissue after the stylet is inserted into an insertion port of a support cylinder at a rear end of the cannula, there is a widely used method comprising the steps of previously boring a hole having the approximately same diameter as an outer diameter of the cannula at a bone cortex of a bone tissue by a drill, and inserting the cannula into the hole bored at the bone cortex of the bone tissue. However, the method also has a fault that a slight space is formed between an outer peripheral surface of the cannula and the bone cortex of the bone tissue in the state that the cannula pierces the bone cortex of the bone tissue. Particularly, when a bone marrow sample is collected by the steps of removing the stylet, attaching an injector to the insertion port of the support cylinder of the cannula and sucking, negative pressure applied to the inside of the bone marrow of the bone tissue leaks due to the slight space. Thus, there is a fault that a bone marrow sample cannot be sufficiently sucked.

The present invention solves the above-described faults of conventional art, and an objective of the present invention is to provide a cannula having a support cylinder which has an insertion port for inserting a stylet and is fixed at a rear end of the cannula, and particularly to provide a cannula which is properly used as a bone marrow biopsy needle for a hard bone tissue such as a jaw bone, can easily carry out an operation for piercing a bone cortex of a bone tissue with weaker force, and gives high airtightness in a state of piercing a bone cortex of a bone tissue.

Present inventors carried out earnest work to solve the above-described problems and, as a result, they found out the followings to complete the present invention. '.A male screw part is formed on an outer periphery of a tip of a cannula, and a cutting edge having a self-tapping function is formed at a tip of the male screw part. Then, a support cylinder is rotated around a center axis of the cannula, while the tip of the cutting edge is contacted to a hole, which is previously bored at a bone cortex of a bone tissue by a drill and has the approximately same diameter as a root diameter of the male screw part of the cannula. By this easy operation, while the cutting edge having a self-tapping function cuts a female screw on a peripheral surface of the hole having the approximately same diameter as the root diameter of the male screw part of the cannula, the cannula can be gradually screwed in until the male screw part pierces the bone cortex of the bone tissue and the tip of the cutting edge reaches a bone marrow of the bone tissue. Therefore, this cannula can be properly used as a bone marrow biopsy needle for a hard bone tissue such as a jaw bone, can easily pierce a bone cortex of a bone tissue with weak force, and can give remarkably high airtightness in a state of piercing a bone cortex of a bone tissue.

According to an aspect of the present invention, a cannula comprises a support cylinder having an insertion port for inserting a stylet and being fixed at a rear end of the cannula, wherein a male screw part is formed on an outer periphery of a tip of the cannula, and a cutting edge, which is made as a recess toward a center axis and has a self-tapping function at one-side end edge of the recess, is formed along an axial direction at the tip side of the male screw part.

Further, the present inventors also found out the followings. In the cannula according to the present invention, when a portion of the male screw, at which the cutting edge is formed, has a shape having a diameter which is gradually reduced toward a tip, an operation of rotating the support cylinder around the center axis of the cannula can be started, while the cutting edge having a self-tapping function is inserted into a hole, which is previously bored at a bone cortex of a bone tissue by a drill and has the approximately same diameter as a root diameter of the male screw part of the cannula. Therefore, operativity can be improved, and thus it is preferable.

Further, they also found out the followings. When plural cutting edges are formed at approximately equal intervals on the outer periphery at the tip side of the male screw part, owing to the incremental number of the cutting edges having a self-tapping function, ability for cutting the female screw on the peripheral surface of the hole previously bored at the bone cortex of the bone tissue is improved. Thus, rotation force, which is required for the operation of screwing the cannula until the male screw part pierces the bone cortex of the bone tissue and the tip of the cutting edge reaches a bone marrow of the bone tissue, can be reduced. Furthermore, when the outer diameter of the male screw part is approximately equal to the diameter of a portion at the rear end side from the male screw part, airtightness in a state of piercing the bone cortex of the bone tissue can be more improved, and thus it is preferable.

Having the above-described constitution, the cannula according to the present invention is properly used as a bone marrow biopsy needle for a hard bone tissue such as a jawbone. Further, a support cylinder is rotated around a center axis of the cannula while the tip of the cutting edge is contacted to a hole, which is previously bored at a bone cortex of a bone tissue by a drill and has the approximately same diameter as a root diameter of the male screw part of the cannula. By this easy operation, while the cutting edge having a self-tapping function cuts a female screw on a peripheral surface of the hole having the approximately same diameter as a root diameter of the male screw part of the cannula, the male screw part of the cannula is engaged with the female screw, which is gradually cut on the peripheral surface of the hole. Thereby, the cannula is gradually advanced and gradually screwed in until the male screw part pierces the bone cortex of the bone tissue and the tip of the cutting edge reaches the bone marrow of the bone tissue. Therefore, the cannula can be screwed in until the tip of the cutting edge reaches the bone marrow of the bone tissue with only rotation force given to the support cylinder around the center axis of the cannula. Thus, the cannula can easily pierce the bone cortex of the bone tissue with weak force. Further, it is not necessary to carry out an operation of pressing a handle of the cannula with strong force in order for the tip of the cannula to pierce the bone cortex of the bone tissue, while this operation is necessary in case of a conventional cannula. Thus, operativity can be remarkably improved in comparison to a conventional cannula, and there is no risk that the cannula is deformed or broken during the operation.

Further, the cannula according to the present invention is gradually screwed into the hole, which is previously bored at the bone cortex of the bone tissue by a drill and has the approximately same diameter as the root diameter of the male screw part of the cannula, until the male screw part pierces the bone cortex of the bone tissue and the tip of the cutting edge reaches the bone marrow of the bone tissue, while the cutting edge having a self-tapping function cuts the female screw on the peripheral surface of the hole having the approximately same diameter as the root diameter of the male screw part of the cannula. Thus, the male screw part is engaged along the peripheral surface of the hole bored at the bone cortex of the bone tissue, when the cannula is screwed in until the male screw part pierces the bone cortex of the bone tissue and the tip of the cutting edge reaches the bone marrow of the bone tissue. Therefore, since a space is not created at all between the cannula and the bone cortex of the bone tissue, airtightness in a state of piercing the bone cortex of the bone tissue can be remarkably enhanced. Further, particularly, when a bone marrow sample is collected by the steps of removing a stylet, attaching an injector to the insertion port of the support cylinder of the cannula, and sucking, negative pressure applied to the inside of the bone marrow of the bone tissue does not leak at all, and thus the bone marrow sample can be efficiently sucked.

Further, as for the cannula according to the present invention, the portion of the male screw where the cutting edge is formed has a shape having a diameter which is gradually reduced toward the tip. Thus, an operation of rotating the support cylinder around the center axis of the cannula can be started, while the cutting edge having a self-tapping function is inserted into a hole, which is previously bored at a bone cortex of a bone tissue by a drill and has the approximately same diameter as a root diameter of the male screw part of the cannula. Therefore, operativity can be improved, and thus it is preferable.

Furthermore, when plural cutting edges are formed at approximately equal intervals on the outer periphery at the tip side of the male screw part, owing to the incremental the number of the cutting edges having a self-tapping function, ability for cutting the female screw on the peripheral surface of the hole previously bored at the bone cortex of the bone tissue is improved. Thus, rotation force, which is required for the operation of screwing the cannula until the male screw part pierces the bone cortex of the bone tissue and the tip of the cutting edge reaches a bone marrow of the bone tissue, can be reduced. Further, spaces formed between the cutting edges, in which cut wastes of the bone cortex cut by the cutting edge are accumulated, can be increased. Thus, the cutting efficiency can be improved. Furthermore, when the outer diameter of the male screw part is approximately equal to the diameter of a portion at the rear end side from the male screw part, airtightness in a state of piercing the bone cortex of the bone tissue can be more improved, and thus it is preferable.
Fig. 1 is a front view to illustrate one example of a cannula according to the present invention.
Fig. 2 is a right side view of Fig. 1.
Fig. 3 is an enlarged front view of A part of Fig. 1.
Fig. 4 is an enlarged left side view of A part of Fig. 1.
Fig. 5 is an enlarged cross sectional view taken along the B-B line of Fig. 4.
Fig. 6 is a front view to illustrate a stylet used with the cannula according to the present invention illustrated in Fig. 1.

A cannula according to the present invention will be described below with reference to the drawings.

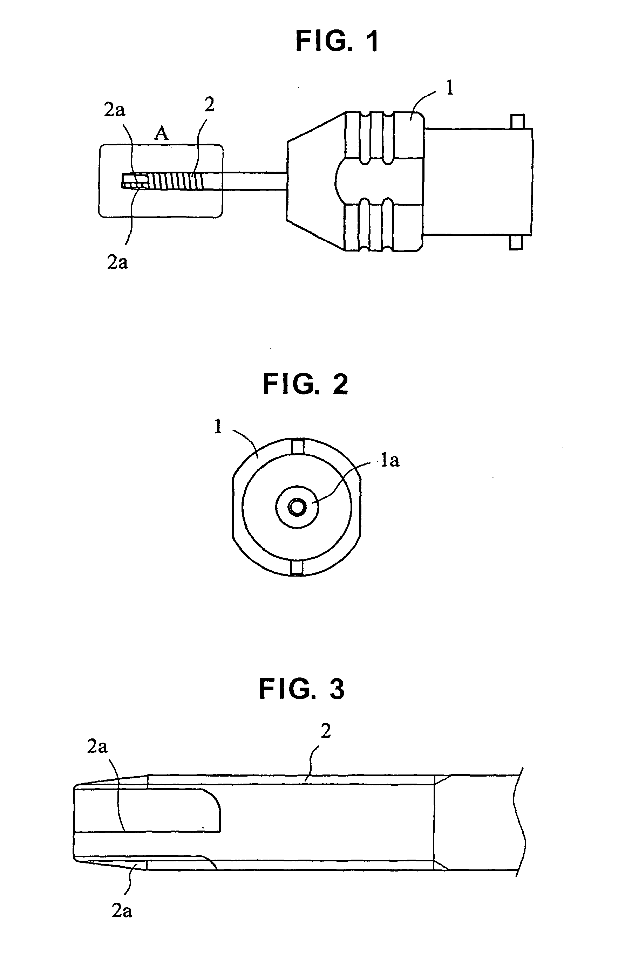

Fig. 1 is a front view to illustrate one example of a cannula according to the present invention.

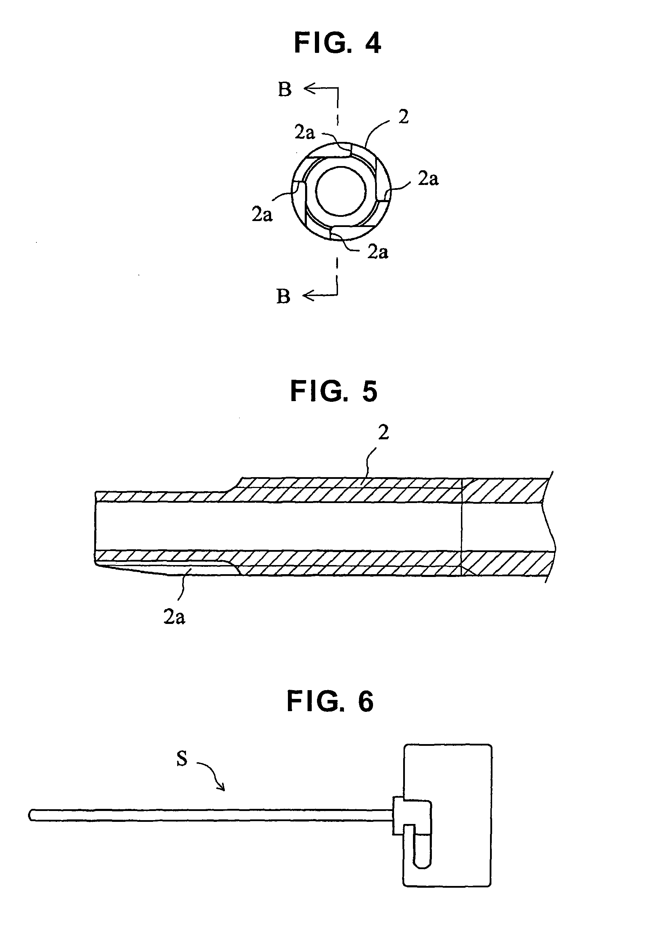

Fig. 2 is a right side view of Fig. 1. Fig. 3 is an enlarged front view of A part of Fig. 1. Fig. 4 is an enlarged left side view of A part of Fig. 1. Fig. 5 is an enlarged cross sectional view taken along the B-B line of Fig. 4. Fig. 6 is a front view to illustrate a stylet used with the cannula according to the present invention illustrated in Fig. 1.

In the drawings, a support cylinder 1 is fixed at a rear end of a cannula and has an insertion port 1a for inserting a stylet S. The support cylinder 1 is given rotation force around a center axis of a cannula according to the present invention by an operator, when the cannula is screwed into a hole, which is previously bored at a bone cortex of a bone tissue and has an approximately same diameter as a root diameter of a male screw part 2 mentioned blow, until the male screw part 2 pierces the bone cortex of the bone tissue and a tip of a cutting edge 2a of the male screw part 2 reaches a bone marrow of the bone tissue. Further, the stylet S or a collection implement is inserted into the insertion port 1a, or a suction implement or an injector is attached to the insertion port 1a.

The support cylinder 1 has the insertion port 1a for inserting the stylet S or the collection implement as illustrated in Fig. 2, and the insertion port 1a of the support cylinder 1 communicates with a hole at a tip of the cylindrical cannula. Thus, the stylet S or the collection implement inserted into the insertionport 1a of the support cylinder 1 is introduced into the cylindrical cannula, or penetrate it to protrude from the hole of the tip of the cylindrical cannula.

The support cylinder 1 can have any shape if having the insertion port 1a for inserting the stylet S. However, for example, when the support cylinder 1 has a shape having a recessed groove on an outer peripheral surface thereof as illustrated in Fig. 1, a shape having a portion to be handled formed in a non-circular shape as illustrated in Figs. 1 and 2, or a shape having levers at both sides (not illustrated), an operator can easily give rotation force around a center axis of the cannula to the support cylinder 1, so that operativity is improved. Thus, it is preferable.

Further, as illustrated in Figs. 1 and 2, projection parts, which are rectangular to the center axis of the cannula, can be provided on an outer peripheral surface of an end part at the opposite side to the cannula of the support cylinder 1, while, as illustrated in Fig. 6, engaging grooves to be engaged with the projection parts of the support cylinder 1 can be formed at a holding part at the rear end side of the stylet S. Thus, the stylet S can be engaged with the support cylinder 1 in a state that the stylet S is inserted into the insertion port 1a of the support cylinder 1. Therefore, for example, when an operator carries out the operation for giving rotation force around the center axis of the cannula, detachment of the stylet S from the cannula can be prevented, and thus it is preferable.

A male screw part 2 is formed on an outer periphery of the tip of the cannula. The male screw part 2 has a cutting edge 2a, which is made as a recess toward the center axis and has a self-tapping function at one-side end edge of the recess, along the axial direction at the tip side of the male screw part 2. The male screw part 2 is gradually screwed into the hole, which is previously bored at the bone cortex of the bone tissue by a drill and has the approximately same diameter as the root diameter of the male screw part 2 of the cannula, by rotation force around the center axis of the cannula being given to the support cylinder 1 while the tip of the cutting edge 2a is contacted to the hole, until the male screw part 2 pierces the bone cortex of the bone tissue and the tip of the cutting edge 2a reaches the bone marrow of the bone tissue. In this case, the male screw part 2 also functions to remarkably increase airtightness in a state of piercing the bone cortex of the bone tissue.

The rotation force around the center axis of the cannula is given to the support cylinder 1 while the tip of the cutting edge 2a formed along the axial direction at the tip side of the male screw part 2 is contacted to the hole, which is previously bored at the bone cortex of the bone tissue by a drill and has the approximately same diameter as the root diameter of the male screw part 2 of the cannula. Then, while the cutting edge 2a, which is made as a recess toward the center axis and has a self-tapping function at one-side end edge of the recess, cuts the female screw on the peripheral surface of the hole having the approximately same diameter as the root diameter of the male screw part 2 of the cannula, the male screw part 2 is engaged with the female screw which is gradually cut on the peripheral surface of the hole. By these steps, the cannula is gradually advanced and screwed in, until the male screw part 2 pierces the bone cortex of the bone tissue and the tip of the cutting edge 2a reaches the bone marrow of the bone tissue.

As the male screw part 2, any screw can be used if being a male screw formed on an outer periphery of the tip of the cannula, that is, a male screw formed on an outer periphery from the middle of the cylindrical cannula to the tip of the cannula. When the outer diameter of the male screw part 2 is approximately equal to the diameter of the portion at the rear end side from the male screw part 2 as illustrated in Figs. 3 to 5, the airtightness in the state of piercing the bone cortex of the bone tissue can be more improved, and thus it is preferable.

The cutting edge 2a, which is made as a recess toward the center axis and has a self-tapping function at one-side end edge of the recessed shape, is formed along the axial direction at the tip side of the male screw part 2. This cutting edge 2a is not limited especially if it can make a female screw, which can be engaged with the male screw part 2, on the peripheral surface of the hole, which is previously bored at the bone cortex of the bone tissue by a drill and has the approximately same diameter as the root diameter of the male screw part 2 of the cannula. However, when the male screw part 2 formed with the cutting edge 2a has a shape having a diameter which is gradually reduced toward the tip as illustrated in Figs 1 and 3 to 5, an operator can start to rotate the support cylinder 1 around the center axis of the cannula while inserting the cutting edge 2a having a self-tapping function into the hole which is previously bored at the bone cortex of the bone tissue by a drill and has the approximately same diameter as the root diameter of the male screw part 2 of the cannula. Thus, operativity can be improved, so it is preferable.

Further, at least one cutting edge 2a is formed. However, when plural cutting edges 2a are formed at approximately equal intervals on an outer periphery at the tip side of the male screw part 2 as illustrated in Figs. 1 and 3 to 5, owing to the incremental number of the cutting edges 2a having a self-tapping function, ability for cutting the female screw on the peripheral surface of the hole previously bored at the bone cortex of the bone tissue by a drill is improved. Thus, rotation force, which is required for the operation of screwing the cannula until the male screw part 2 pierces the bone cortex of the bone tissue and the tip of the cutting edge 2a reaches a bone marrow of the bone tissue, can be reduced. So, it is preferable. Further, a space formed between the cutting edges 2a, in which cut wastes of the bone cortex cut by cutting edges 2a are accumulated, can be increased. Thus, the cutting efficiency can be improved, so it is preferable.

Then, a using method of a cannula according to the present invention having the above-described constitution will be described.

As a preparatory step, a hole having the approximately same diameter as the root diameter of the male screw part of the cannula is bored at the bone cortex of the bone tissue by a drill, at which a bone marrow sample is collected or various kinds of medical agents are injected. Further, the stylet S is inserted into the insertion port 1a of the support cylinder 1 of the cannula so as to carry out an operation for mounting the stylet S on the cannula.

Then, the support cylinder 1 is rotated around the center axis of the cannula while the tip of the cutting edge 2a is contacted to the hole bored at the bone cortex of the tissue. Thereby, the cannula is gradually screwed in until the male screw part 2 pierces the bone cortex of the bone tissue and the tip of the cutting edge 2a reaches the bone marrow of the bone tissue.

By this operation, the cannula can be gradually advanced according to screwing of the male screw part 2 of the cannula into the female screw which is gradually cut on the peripheral surface of the hole bored at the bone cortex of the tissue, while the cutting edge 2a having a self-tapping function cuts the female screw on the peripheral surface of the hole. Thus, the cannula can be screwed in until the tip of the cutting edge 2a reaches to the bone marrow of the bone tissue, by only rotation force around the center axis of the cannula being given to the support cylinder 1.

At this time, when the male screw part 2 formed with the cutting edge 2a has a shape having a diameter which is gradually reduced toward the tip as illustrated in Figs 1 and 3 to 5, the support cylinder 1 can be started to be rotated around the center axis of the cannula while the cutting edge 2a having a self-tapping function is inserted into the hole which is previously bored at the bone cortex of the bone tissue by a drill and has the approximately same diameter as the root diameter of the male screw part 2 of the cannula. Thus, operativity can be improved, so it is preferable.

As described above, when the cannula according to the present invention is screwed in until the male screw part 2 pierces the bone cortex of the bone tissue and the tip of the cutting edge 2a reaches the bone marrow of the bone tissue, the male screw part 2 is engaged along the peripheral surface of the hole bored at the bone cortex of the bone tissue. Thus, since a space is not created at all between the cannula and the bone cortex of the bone tissue, high airtightness in a state of piercing the bone cortex of the bone tissue can be obtained.

Further, after the cannula according to the present invention pierces the bone cortex of the bone tissue with high airtightness, a bone marrow sample can be collected from the bone tissue, or various kinds of medical agents can be injected into a bone marrow of the bone tissue.

## Claims

1. A cannula comprising a support cylinder (1) having an insertion port (1a) for inserting a stylet (S) and being fixed at a rear end of the cannula, wherein a male screw part (2) is formed on an outer periphery of a tip of the cannula, and a cutting edge (2a), which is made as a recess toward a center axis and has a self-tapping function at one-side end edge of the recess, is formed along an axial direction at the tip side of the male screw part (2).

2. The cannula as claimed in claim 1,
wherein a portion of the male screw part (2), at which the cutting edge (2a) is formed, has a shape having a diameter gradually reduced toward a tip.

3. The cannula as claimed in claim 1 or 2,
wherein plural cutting edges (2a) are formed at approximately equal intervals on an outer periphery at the tip side of the male screw part (2).

4. The cannula as claimed in any one of claims 1 to 3,
wherein an outer diameter of the male screw part (2) is approximately equal to a diameter of a portion at the rear end side from the male screw part (2).
